# EUROPEAN PATENT APPLICATION

(11) **EP 4 559 391 A1**
(43) Date of publication of application: **28.05.2025**
(21) Application number: 24214503.5
(22) Date of filing: 21.11.2024
(51) Int. Cl.: A61B 5/145, A61B 5/00

(54) **METHOD FOR PROVIDING ALARM BASED ON BIOMETRIC DATA**

(30) Priority: 22.11.2023 KR 20230163704
(71) Applicant: I-SENS, INC., Seoul 06646 (KR)
(72) Inventor: CHO, Hee Gyung, 06646 Seoul (KR)
(74) Representative: BCKIP Part mbB

(57) **Abstract**

The present disclosure relates to a method for providing an alarm based on biometric data. More specifically, the present disclosure relates to a method for providing an alarm, in which when a set alarm generation condition is satisfied, in a set alarm period, an alarm form is changed at predetermined intervals, so that a user may be induced to cope with an alarm situation by continuously recognizing an alarm, and in which when a condition for an important alarm such as an acute hypoglycemia alarm occurs, the alarm is provided to the user in an emergency alarm form accurately recognizable by the user irrespective of an operation mode of a receiver or a set alarm form, so that the user may rapidly cope with acute hypoglycemia to prevent being in danger.

## Description

### CROSS-REFERENCE TO RELATED APPLICATION(S)

This application claims the benefit of Korean Patent Application No. 10-2023-0163704, filed on November 22, 2023, in the Korean Intellectual Property Office.

### TECHNICAL FIELD OF THE INVENTION

The present disclosure relates to a method for providing an alarm based on biometric data. More specifically, the present disclosure relates to a method for providing an alarm, in which when a set alarm generation condition is satisfied, in a set alarm period, an alarm form is changed and the alarm is provided at predetermined intervals, so that a user may be induced to cope with an alarm situation by continuously recognizing the alarm, and in which when a condition for an important alarm such as an acute hypoglycemia alarm occurs, the alarm is provided to the user in an emergency alarm form accurately recognizable by the user irrespective of an operation mode of a receiver or a set alarm form, so that the user may rapidly cope with acute hypoglycemia to prevent being in danger.

### BACKGROUND

Diabetes is a global cause of death and a factor causing a physical disability. Accordingly, many people have a health problem due to diabetes. Particularly, diabetes is a serious disease causing heart and renal diseases, blindness, neural damage, and high blood pressure. Long-time clinical researches show that induction of a complication may be greatly reduced by moderately adjusting a blood sugar value. Thus, continually controlling diabetes is important. An important factor therefor is self-monitoring of the blood sugar value.

A self-bio-measuring device with which a user may directly examine blood sugar of the user is widely distributed and used for such demand. A general blood sugar meter measures the blood sugar value of the user by putting blood of the user on a sensor strip that is an examination paper. In other words, the sensor strip on which the blood is put is inserted, and the blood sugar value measured through the sensor strip is displayed.

At this point, the blood sugar meter receives an electrical signal generated by an electrochemical reaction of the blood which is gathered and a reaction substance in the sensor strip, and the blood sugar value is measured. Such a blood gathering-type blood sugar meter that uses a finger prick method helps control of blood sugar of a diabetes patient. However, accurately identifying the blood sugar value which frequently changes is difficult because the blood gathering-type blood sugar meter shows only a result at a time of measuring.

The diabetes patient generally alternates between a hyperglycemia state and a hypoglycemia state. An emergency may occur in the hypoglycemia state, and the patient may lose consciousness or may die at worst when supply of sugar does not last for a long time. Thus, immediately discovering the hypoglycemia state is greatly important for the diabetes patient. However, a blood gathering-type blood sugar meter that intermittently measures the blood sugar has a clear limitation.

In order to overcome the limitation on such a blood gathering-type blood sugar meter, a continuous glucose monitoring system (CGMS) that is inserted into a human body to measure blood sugar at intervals of several minutes has been developed. Control for the diabetes patient and coping with the emergency may be facilitated with the continuous glucose monitoring system.

The continuous glucose monitoring system includes a body-attachable unit that is inserted into the human body to measure the blood sugar from a body fluid of a user and a receiver that is in communication with the body-attachable unit to display blood sugar information measured by the body-attachable unit.

The receiver may output, to the user, the blood sugar information which is received from the body-attachable unit and also provide an alarm to the user based on the blood sugar information when the user has hypoglycemia and hyperglycemia or monitor a use state of the body-attachable unit to provide the alarm to the user.

For example, when a blood sugar value of the user measured through the body-attachable unit shows hypoglycemia, the receiver may provide the alarm to the user to prevent the user from being in the emergency.

Also, the receiver may monitor a use period of the body-attachable unit to provide an alarm for replacement of the body-attachable unit or provide an alarm for requesting correction of the body-attachable unit.

Meanwhile, in order to provide accurate blood sugar information to the user, the blood sugar information received from the body-attachable unit may be corrected with an additional blood sugar meter at an initial stage and then continuously corrected, at predetermined intervals in the use period of the body-attachable unit, to be a reference blood sugar value measured by the additional blood sugar meter. When there is not correction for the blood sugar value which measured by the body-attachable unit, reliability of the blood sugar value measured by the body-attachable unit is decreased. Thus, an alarm for periodically correcting the blood sugar value may be provided to the user.

As such, the receiver may output various alarms to the user during use of the body-attachable unit. In a case of an important alarm, an additional alarm may be provided to the user when the user does not respond to the alarm after whether the user performs a required action in response to the alarm or inputs required information is determined.

In the related art, a snooze function of repeating and providing an alarm to a user at predetermined intervals in an alarm period after an alarm is generated and before an alarm cancelation order is input is provided.

However, the snooze function in the related art is to simply provide the alarm before the alarm cancelation order is input from the user. Since the snooze function in the related art repeats the alarm regardless of whether an alarm cancelation condition has been satisfied (for example, the user has performs the required action or input the information) in response to the alarm, although the alarm cancelation condition has been satisfied, the alarm is repeated and provided to the user. Thus, the user feels inconvenience.

Furthermore, since the snooze function in the related art repeats and provides alarms in an identical form to the user at predetermined time intervals, the user is accustomed to the repeating alarms to ignore or not recognize the alarm.

Also, the snooze function in the related art repeats and provides the alarms in the identical form to the user even if the user has recognized the alarms. Through this, the user feels fatigue due to the repeated alarms or feels inconvenience as the alarms are repeatedly output even when the user is reluctant to reveal use of the body-attachable unit to surrounding people or does not want to be disturbed due to the alarms during a meeting.

### SUMMARY OF THE INVENTION

An aspect provides an alarm to a user while an alarm form is changed at predetermined intervals in an alarm period when a set alarm generation condition is satisfied.

Another aspect also provides a method of determining whether an alarm cancelation condition is satisfied at a time at which the alarm is generated by changing the alarm form at the predetermined intervals as the alarm generation condition is satisfied and automatically stopping generation of the alarm even before an additional alarm cancelation order from the user is input when the alarm cancelation condition is satisfied.

Still another aspect also provides the alarm by determining an operation mode of a receiver when the alarm generation condition is satisfied and changing a set alarm form to an alarm form corresponding to the operation mode of the receiver according to the alarm generation condition.

Still another aspect also provides, when a condition for an important alarm such as an acute hypoglycemia alarm occurs, the alarm in an emergency alarm form, with which the user may accurately recognize the alarm, irrespective of the operation mode of the receiver or the set alarm form.

According to an aspect, there is provided a method for providing an alarm for continuous biometric data, the method including determining whether a set alarm generation condition is satisfied, and based on that the alarm generation condition is satisfied, generating an alarm by modifying the alarm form during a set alarm period, and sequentially outputting the generated alarm to a user.

The outputting of the alarm to the user may include outputting a first form alarm by generating the first form alarm based on that the alarm generation condition is satisfied, determining whether an alarm stop order is input or whether an alarm activation time for the first form alarm is ended, stopping output of the first form alarm, which is currently output, based on that the alarm stop order is input or based on that the alarm activation time for the first form alarm is ended, determining whether an alarm cancelation condition is satisfied after the output of the first form alarm is stopped, and based on that the alarm generation condition is not satisfied, outputting, to the user, an alarm in a form is different from that of the first form alarm by generating the alarm in the form is different from that of the first form alarm.

The alarm in the changed form may be generated to be sequentially output to the user before an alarm cancelation order is input in the alarm period.

The alarm in the changed form may be generated to be sequentially output to the user until the alarm cancelation condition is satisfied in the alarm period.

The alarm form which is sequentially changed in the alarm period may be sequentially changed and generated depending on a predetermined alarm form, or the alarm form which is sequentially changed in the alarm period may be randomly set and sequentially changed and generated.

The method may further include determining an operation mode of a receiver at a time point of generating the alarm, and the alarm may be generated and output in a form conforming with the operation mode of the receiver.

The method comprises determining, at a time point of generating the alarm, whether the alarm generation condition is a first alarm generation condition, and based on that the alarm generation condition which is determined at the time point of generating the alarm is the first alarm generation condition, the alarm may be output in a form of an emergency alarm disregarding of the set alarm form or an operation mode of a receiver.

The first alarm generation condition may be an alarm generation condition that the biometric data shows acute hypoglycemia.

Whether the alarm generation condition is a second alarm generation condition and whether a change rate of the biometric data exceeds a threshold change rate may be simultaneously determined, and based on that the change rate of the biometric data exceeds the threshold change rate while the alarm generation condition is the second alarm generation condition, the alarm may be output in a form of an emergency alarm disregarding of the set alarm form or an operation mode of a receiver.

The second alarm generation condition may be an alarm generation condition that the biometric data shows hypoglycemia or hyperglycemia
Additional aspects of example embodiments will be set forth in part in the description which follows and, in part, will be apparent from the description, or may be learned by practice of the disclosure.

According to example embodiments, when a set alarm generation condition is satisfied, an alarm form may be changed at predetermined intervals, and an alarm may be provided during a set alarm period. Through this, a user may be induced to cope with an alarm situation by continuously recognizing the alarm.

According to example embodiments, when an alarm generation condition is satisfied, the alarm is generated by changing the alarm form at the predetermined intervals. Whether an alarm cancelation condition is satisfied may be determined at an alarm generation time point, and when the alarm cancelation condition is satisfied, alarm generation is automatically stopped even before an additional alarm cancelation order from the user is input, so that the user is not to feel inconvenience.

According to example embodiments, when the alarm generation condition is satisfied, an operation mode of the receiver is determined, so that the set alarm condition is changed to an alarm form corresponding to the operation mode of the receiver. Through this, it is possible to prevent occurrence of an alarm in a form not intended by the user.

According to example embodiments, when a condition for an important alarm such as an acute hypoglycemia alarm occurs, the alarm is provided to the user in an emergency form accurately recognizable by the user irrespective of the operation mode of the receiver or the set alarm form. Through this, the user may rapidly cope with acute hypoglycemia to prevent being in danger.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 is a diagram for describing a system for measuring continuous biometric data according to the present disclosure;
FIG. 2 is a function block diagram for describing a device providing an alarm according to the present disclosure;
FIG. 3 is a function block diagram for describing an example of an alarm generation unit according to the present disclosure;
FIG. 4 is a flowchart for describing a method of generating an alarm based on biometric data according to the present disclosure;
FIG. 5 illustrates an example of an alarm form combination that is set in the present disclosure;
FIG. 6 is a diagram for describing an example of a user interface screen for setting an alarm form;
FIG. 7 is a diagram for describing an example of a generation condition, by alarm, for an alarm pattern combination in the present disclosure;
FIG. 8 is a flowchart for describing an example of providing an alarm according to a receiver operation mode in a method of providing the alarm according to the present disclosure;
FIG. 9 illustrates an example of a mapping table at a time of changing an alarm form so that the alarm form conforms with an operation mode of a receiver;
FIG. 10 is a flowchart for describing an example of providing an alarm when an emergency alarm is generated in a method of providing the alarm according to the present disclosure; and
FIG. 11 is a diagram for describing an example of a generation condition, by alarm, for an emergency alarm pattern combination in the present disclosure.

### DETAILED DESCRIPTION OF EXEMPLARY EMBODIMENTS

Technical terms used in the present disclosure is merely to describe predetermined example embodiments. It should be noted that the technical terms is not to limit the present disclosure. Also, the technical terms used in the present disclosure should be construed, unless the terms are specially defined to have other meanings, as having meanings generally understood by those skilled in the art to which the present disclosure belong and should not be construed as having excessively inclusive meanings and excessively narrow meanings. In addition, when being wrong technical terms not accurately describing the idea of the present disclosure, the technical terms used in the present disclosure is to be substituted with technical terms understandable by those skilled in the art.

Also, terms in a singular form used in the present disclosure include terms in a plural form unless an apparently and contextually conflicting description is present. In the present disclosure, terms such as "including" or "comprising" should not be construed as that various elements or various operations described in the present disclosure are necessarily included. The terms should be construed as that some of the elements or the operations may not be included or that additional elements or operations may be further included.

Furthermore, the accompanying drawings are merely to easily understand the idea of the present disclosure. It should be noted that the idea of the present disclosure is not to be construed as being limited by the accompanying drawings.

Hereinafter, a method for providing an alarm based on biometric data according to the present disclosure will be described with reference to the accompanying drawings.

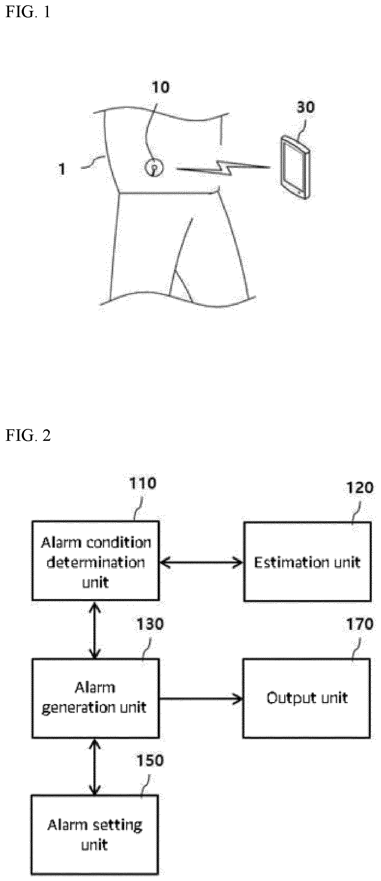

FIG. 1 is a diagram for describing a system for measuring continuous biometric data according to the present disclosure.

Referring to FIG. 1, the system for measuring the continuous biometric data according to an example embodiments of the present disclosure includes a body-attachable unit 10 and a receiver 30.

The body-attachable unit 10 is attached to a body 1. When the body-attachable unit 10 is attached to the body 1, an end of a sensor of the body-attachable unit 10 is inserted into skin to continuously measure the biometric data such as blood sugar from a body fluid of a human body. Here, the body-attachable unit 10 is formed of the sensor which is inserted into the skin to measure the biometric data and a transmitter that transmits the measured biometric data to the receiver 30. The sensor and the transmitter are assembled together to form the body-attachable unit 10, and the body-attachable unit 10 is mounted to an applicator (not illustrated) that attaches the body-attachable unit 10 to the body 1.

A user may take out the applicator from packaging, position the applicator at a position at which the body-attachable unit 10 is to be mounted, and then attach the body-attachable unit 10 to the skin with the applicator.

After the body-attachable unit 10 is attached to the skin, the body-attachable unit 10 and the receiver 30 are in communication with and connected to each other to transmit and receive the biometric data measured by the body-attachable unit 10.

Here, the receiver 30 may connect communication with the body-attachable unit 10 and may use an exclusive terminal, a smartphone, or the like receiving the biometric data from the body-attachable unit 10 to provide the biometric data to the user. The receiver 30 is not limited to the smartphone, a tablet personal computer (PC), a laptop, or the like. Any terminal may be used as long as the terminal includes a communication function and a program or an application may be installed in the terminal.

After being in communication with and connected to the body-attachable unit 10, the receiver 30 periodically receives the biometric data from the body-attachable unit 10 and outputs, to the user, biometric data generated by correcting the received biometric data.

The receiver 30 provides an alarm to the user based on a use state of the body-attachable unit 10 or provides the alarm to the user based on the biometric data received from the body-attachable unit 10.

For example, when the biometric data exceeds a set value, for example, when a blood sugar state of the user is hypoglycemia, acute hypoglycemia, or hyperglycemia, the receiver 30 provides an alarm for notifying the user of the blood sugar state based on the biometric data received from the body-attachable unit 10. Alternatively, the receiver 30 provides a replacement alarm to the user when a time to replace the body-attachable unit 10 which has a limited use time arrives or provides a correction alarm to the user when a time to input correction information on the biometric data measured through the body-attachable unit 10 arrives.

A variety of biometric data on the user may be continuously measured through the body-attachable unit 10. However, blood sugar information will be hereinafter described as an example of the biometric data.

FIG. 2 is a function block diagram for describing a device providing an alarm according to the present disclosure.

In specific description with reference to FIG. 2, an alarm condition determination unit 110 determines whether an alarm generation condition has been satisfied based on blood sugar information measured by a body-attachable unit or a use state of the body-attachable unit, determines whether an alarm cancelation condition has been satisfied based on the blood sugar information measured by the body-attachable unit or the use state of the body-attachable unit, or determines whether the alarm cancelation condition is satisfied based on a user order that is input through a user interface (not illustrated).

When the alarm generation condition is satisfied, an alarm generation unit 130 activates an alarm to sequentially generate, while changing a form of the alarm, the alarm which has the changed form during a set alarm period. The alarm generation unit 130 sequentially generates alarms in different forms until an alarm cancelation order is input in the alarm period and stops alarm generation when the alarm cancelation order is input.

The alarm generation unit 130 determines whether the alarm cancelation condition is satisfied at a time of generating the alarms in the different forms. When the alarm cancelation condition is satisfied at the time of generating the alarms, the alarm generation unit 130 does not generate the alarm any further by automatically canceling the alarm even if an additional alarm cancelation order is input.

An alarm setting unit 150 may set an alarm form to sequentially generate according to an alarm setting order that is input through the user interface and may store the set alarm form. Desirably, the alarm form may be set differently for each alarm type.

An output unit 170 outputs alarms in various forms, which are generated by the alarm generation unit 130, to a user. Here, a speaker or a display may be used as the output unit 170. The output unit 170 may output the alarm as audio to the user through the speaker or output the alarm as an image to the user through the display.

Here, various types of alarms set by the user or a manufacturer of the body-attachable unit and respective alarm generation conditions of the alarms may be different from each other.

As an example of a set alarm and an alarm generation condition for each alarm, when the body-attachable unit has a limited use period, and when a replacement period of the body-attachable unit arrives within a set time, the alarm generation condition is satisfied. When a blood sugar value measured by the body-attachable unit exceeds a set threshold blood sugar value, the alarm generation condition is satisfied. When a correction period of the body-attachable unit arrives within a set time, the alarm generation condition is satisfied.

Here, the user may set a first threshold blood sugar value showing hyperglycemia, set a second threshold blood sugar value showing hypoglycemia, or set a third threshold blood sugar value showing acute hypoglycemia. When a blood sugar value of the user measured through the body-attachable unit exceeds the set threshold blood sugar value, an alarm generation condition for hyperglycemia, hypoglycemia, or acute hypoglycemia is satisfied. Here, a threshold blood sugar value for at least one of hyperglycemia, hypoglycemia, or acute hypoglycemia may be predetermined by the manufacturer. Desirably, the threshold blood sugar value for at least one of hyperglycemia, hypoglycemia, or acute hypoglycemia may be predetermined by the manufacturer and may be allowed to be changed or not changed by the user.

As another example of the alarm generation condition, when a future blood sugar value is estimated at a current time point, and when the estimated blood sugar value exceeds the first threshold blood sugar value, the second threshold blood sugar value, and the third threshold blood sugar value, the alarm generation condition is satisfied.

An alarm provision device according to the present disclosure may further include an estimation unit 120 in order to estimate the future blood sugar value.

The estimation unit 120 may use a past blood sugar value in a predetermined past period from the current time point to estimate, with extrapolation, the estimated blood sugar value which is at a future time point. In order to estimate the estimated blood sugar value from the past blood sugar value, various estimation algorithms such as a polynomial regression algorithm, an autoregressive algorithm, and a neural network-based mapping algorithm may be used. Here, the future time point may be a future time point at which a time such as five minutes, ten minutes, thirty minutes, or an hour elapses from the current time point.

Further desirably, the estimation unit 120 may calculate a range of a changeable estimated blood sugar value with physiological information on the user such as an age, gender, a height, a weight, and an amount of body fat of the user and may verify the estimated blood sugar value based on whether the estimated blood sugar value is within the range of the estimated blood sugar value. In other words, when being within the range of the blood sugar value, the estimated blood sugar value is estimated to be the estimated blood sugar value. However, when the estimated blood sugar value is out of the range of the estimated blood sugar value, the estimated blood sugar value is calculated as being within a maximum range of the estimated blood sugar value. Thus, although the estimated blood sugar value is out of the range of the estimated blood sugar value, the estimated blood sugar value may be limited within a maximum available range. Through this, an estimated value having a large error due to noise or the like may be prevented from being calculated.

Meanwhile, for example, the alarm cancelation condition is satisfied when the body-attachable unit is replaced with a new body-attachable unit in a case in which the replacement period of the body-attachable unit arrives. The alarm cancelation condition is satisfied when the user injects insulin or the like or has a meal after the blood sugar value estimated by the body-attachable unit exceeds the set threshold blood sugar value. The alarm cancelation condition is satisfied when information on a reference blood sugar value that the user measures with a blood sugar meter as the replacement period of the body-attachable unit arrives is input.

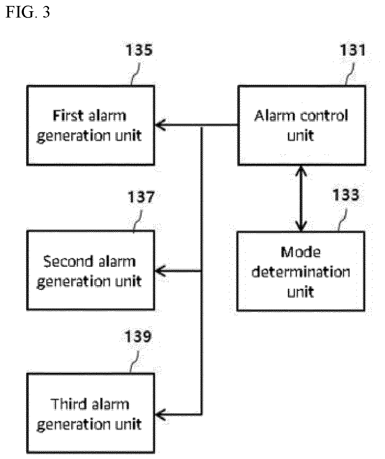

The alarm generation unit 130 according to the present disclosure sequentially changes the alarm form at intervals of a predetermined time in the set alarm period while maintaining an alarm activation state for the alarm period when the alarm generation condition is satisfied, and FIG. 3 is a function block diagram for describing an example of the alarm generation unit according to the present disclosure.

In specific description with reference to FIG. 3, when a set alarm generation condition is satisfied, an alarm control unit 131 determines an alarm form combination mapped to an alarm type and generates an alarm generation signal according to the determined alarm form combination.

A first alarm generation unit 135 generates a signal for generating a first form alarm. A second alarm generation unit 137 generates a signal for generating a second form alarm.

A third alarm generation unit 139 generates the signal for generating the third form alarm. The alarm form combination according to the alarm type is formed in combination of at least one of the first form alarm, the second form alarm, and the third form alarm, but the alarm form combination may be formed of a different number of alarms depending on a field to which the present disclosure is applied.

Meanwhile, generation conditions for the first form alarm, the second form alarm, and the third form alarm which form the alarm form combination according to the alarm type may be set to be different from each other. For example, a generation condition such as an alarm activation time, an alarm stop time, and alarm intensity may be set to be different from another.

The alarm control unit 131 may sequentially generate a first alarm generation signal for generating the first form alarm according to a generation condition for the first form alarm, a second alarm generation signal for generating the second form alarm according to a generation condition for the second form alarm, and a third alarm generation signal for generating the third form alarm according to a generation condition for the third form alarm.

When receiving the first alarm generation signal from the alarm control unit 131, the first alarm generation unit 135 generates the first form alarm according to the first alarm generation signal and outputs the generated first form alarm to the user through an output unit.

When receiving the second alarm generation signal from the alarm control unit 131, the second alarm generation unit 137 generates the second form alarm according to the second alarm generation signal and outputs the generated second form alarm to the user through the output unit.

When receiving the third alarm generation signal from the alarm control unit 131, the third alarm generation unit 139 generates the third form alarm according to the third alarm generation signal and outputs the generated third form alarm to the user through the output unit.

When an alarm generation condition is satisfied, the alarm control unit 131 sequentially generates the alarm generation signal through at least one of the first alarm generation unit 135, the second alarm generation unit 137, and the third alarm generation unit 139 according to the alarm form combination mapped to the alarm type. When an alarm stop order for stopping an alarm that is currently output is input through the user interface, or when an alarm activation time of the alarm that is currently output is ended, the alarm that is currently output is stopped. After a set time interval elapses since the alarm stops, a next alarm in a different form is continuously generated according to the alarm form combination.

Desirably, the alarm control unit 131 sequentially changes and generates alarms in different forms according to the alarm form combination and determines whether an alarm cancelation condition is satisfied at each time point of changing and generating an alarm form. When the alarm cancelation condition is satisfied at a time point of changing and generating the alarm form, an alarm in a next form may not be generated even if an additional alarm cancelation order is not input.

Meanwhile, the alarm generation unit may further include a mode determination unit 133. The mode determination unit 133 determines an operation mode of a receiver at time points of generating the first form alarm, the second form alarm, and the third form alarm. The alarm control unit 131 is synchronized with the operation mode of the receiver at the time points of generating the first form alarm, the second form alarm, and the third form alarm to change the first form alarm, the second form alarm, and the third form alarm, which are predetermined, so that the first form alarm, the second form alarm, and the third form alarm conform with the operation mode of the receiver. The alarm control unit 131 also generates the first alarm generation signal, the second alarm generation signal, and the third alarm generation signal so that the first form alarm, the second form alarm, and the third form alarm are generated in a mode identical to the operation mode of the receiver.

Desirably, the alarm control unit 131 determines whether the alarm generation condition is an emergency alarm generation condition at a time of generating an alarm. When the alarm generation condition is the emergency alarm generation condition, the alarm may be output in a form of an emergency alarm irrespective of the set alarm form and the operation mode of the receiver.

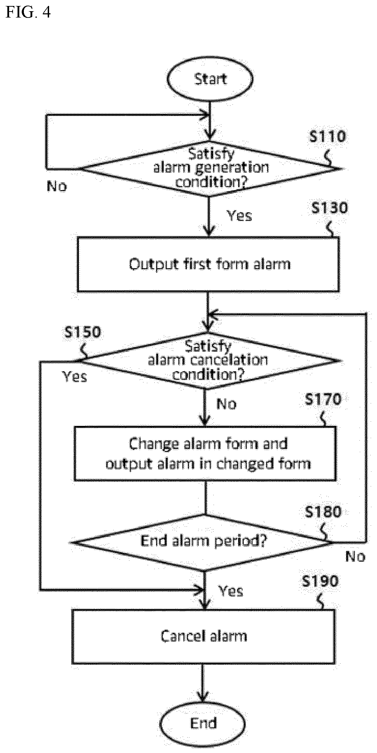

FIG. 4 is a flowchart for describing a method of generating an alarm based on biometric data according to the present disclosure.

In specific description with reference to FIG. 4, whether an alarm generation condition is satisfied is determined in operation S 110.

When an alarm generation condition is satisfied, an alarm period, an alarm form combination, and a generation condition for each alarm according to an alarm type is determined. To begin with, an alarm form and a generation condition of the first form alarm are determined in the alarm form combination, so that a first alarm generation signal is generated, and the first form alarm is generated according to the first alarm generation signal and output to a user in operation S130. Here, a generation condition for the first form alarm may be an activation time of the first form alarm, a stop time of the first form alarm, and intensity of the first form alarm. For example, the first form alarm is generated in set alarm intensity for an activation time and stops being generated for a stop time after the activation time elapses. Even before the activation time of the first form alarm elapses, when an alarm stop order is input, generation of the first form alarm may be ended.

After the stop time of the first form alarm elapses, an alarm form of a second form alarm, an activation time, a stop time, and alarm intensity of the second form alarm are determined, so that a second alarm generation signal is generated in operation S170. The second form alarm is generated in set intensity for the activation time according to the second alarm generation signal to be provided to the user through the output unit 170 and stops being generated for the stop time after the activation time elapses. Even before the activation time of the second form alarm elapses, when the alarm stop order is input, generation of the second form alarm may be ended.

Desirably, in operation S 150, whether an alarm cancelation condition is satisfied may be determined at a time of generating the second form alarm after the stop time of the first form alarm elapses. When the alarm cancelation condition is satisfied at the time of generating the second alarm form, although an additional alarm cancelation order is not input, an alarm is canceled in operation S 190 so that a generation signal for an alarm in another form in the alarm form combination except the first form alarm is not additionally generated.

After the stop time of the second form alarm elapses, whether the alarm period is ended or whether an alarm in a form to be additionally generated remains in the alarm form combination is determined in operation S 180. When the alarm period is not ended, or when the alarm in the form to be additionally generated remains, whether the alarm cancelation condition is satisfied is determined at a time of generating the third form alarm.

Operation S 150 to operation S 180 which are described above are repeated before the alarm period is ended or until the alarm in the form to be additionally generated remains in the alarm form combination. Whether the alarm cancelation condition is satisfied is determined at each alarm generation time point is determined, and although the additional alarm cancelation order is not input at an alarm generation time point, an alarm corresponding to a corresponding alarm generation condition is canceled so that an alarm generation signal is not additionally generated.

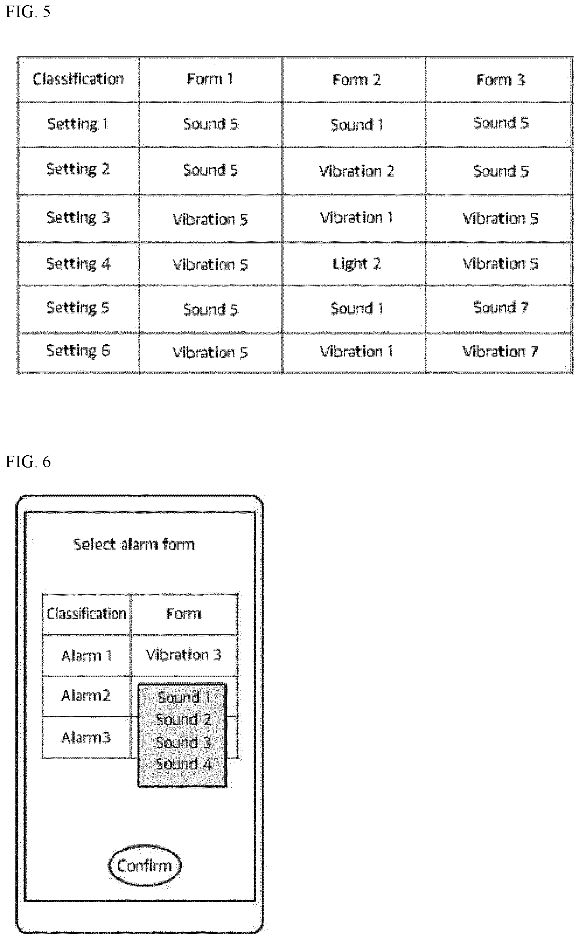

FIG. 5 illustrates an example of an alarm form combination that is set in the present disclosure.

As illustrated in FIG. 5, when the alarm form combination is formed of three alarm forms, setting 1 is formed in combination of sound 5, sound 1, and sound 5. Setting 2 is formed in combination of sound 5, vibration 2, and sound 5. Setting 3 is formed in combination of vibration 5, vibration 1, and vibration 5. Setting 4 is formed in combination of vibration 5, sound 2, and vibration 5. Setting 5 is formed in combination of sound 5, sound 1, and sound 7. Setting 6 is formed in combination of vibration 5, vibration 1, and vibration 7.

Here, setting 1 to setting 6 may be preset by a manufacturer, or the three alarm forms which form the alarm form combination may be set by selection by a user.

Modes of the three alarm forms forming the alarm form combination may be set to be a sound, vibration, light, and the like. Intensity or a frequency may set differently depending on alarm forms by sound, vibration, or light.

FIG. 6 is a diagram for describing an example of a user interface screen for setting an alarm form.

As illustrated in FIG. 6, a user may select a first alarm form, a second alarm form, and a third alarm form for each alarm type to set an alarm form combination. Desirably, a user interface is activated so that intensity or a frequency of the second alarm form may be selected only in a range smaller than that of the first alarm form, and the user interface is activated so that intensity or a frequency of the third alarm form may be selected only in a range equal to or larger than that of the first alarm form.

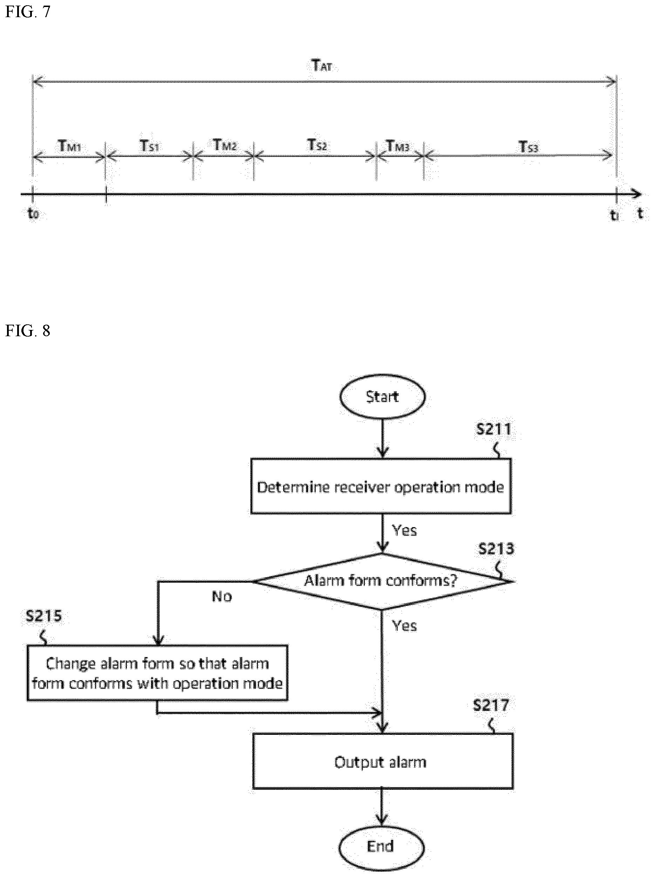

FIG. 7 is a diagram for describing an example of a generation condition, by alarm, for an alarm pattern combination in the present disclosure.

As illustrated in FIG. 7, alarms in different forms are sequentially provided to a user in an alarm period TAT of an alarm pattern combination that is set for each alarm type. A first form alarm is generated to be a generation condition of a first activation time TM1 and a first stop time TS1, is output to the user for the first activation time TM1, and then stops being output for the first stop time TS1.

A second form alarm is generated to be a generation condition of a second activation time TM2 and a second stop time TS2, is output to the user for the second activation time TM2, and then stops being output for the second stop time TS2.

A third form alarm is generated to be a generation condition of a third activation time TM3 and a third stop time TS3, is output to the user for the third activation time TM3, and then stops being output for the third stop time TS3.

An activation time and a stop time of each alarm pattern forming the alarm pattern combination for each alarm type may be equal to each other. However, desirably, depending on a generation order of alarm patterns, the activation time may be decreased, and the stop time may be increased. As such, by decreasing the activation time and increasing the stop time depending on the generation order of the alarm patterns, fatigue of the user due to repeated alarms may be reduced while an alarm may be continually recognized by the user.

FIG. 8 is a flowchart for describing an example of providing an alarm according to a receiver operation mode in a method of providing the alarm according to the present disclosure.

Referring to FIG. 8, in operation S211, a set operation mode of a receiver is determined at a time point of generating the alarm as an alarm generation condition is satisfied.

In operation S213, whether a first form alarm and the operation mode of the receiver conform with each other in an alarm form combination is determined.

In operation S217, when the first form alarm and the operation mode of the receiver conform with each other, a first alarm generation signal is generated to be the first form alarm, which is predetermined, and output to a user.

However, when the first form alarm and the operation mode of the receiver are different from each other, in operation S215, the first alarm generation signal is generated by changing the predetermined first form alarm so that the first form alarm conforms with the operation mode of the receiver, and in operation S217, a first alarm is output to the user according to the generated first alarm generation signal. That is, when the first form alarm is set to be a sound, and when the operation mode of the receiver is set to be vibration at a time point of generating an alarm generation signal for the first form alarm, the alarm generation signal is generated to be the vibration by changing the first form alarm so that the first form alarm conforms with the operation mode of the receiver.

As such, whether a predetermined second form alarm or a predetermined third form alarm conforms with the operation mode of the receiver is determined at a time point of generating the second form alarm or the third form alarm, an operation mode of the second form alarm or the third form alarm and the operation mode of the receiver are synchronized with each other, and the second alarm generation signal or the third alarm generation signal is generated.

As such, the operation mode of the receiver is determined at an alarm generation time point, so that an alarm form is changed to conform with the operation mode of the receiver. Through this, an alarm against an intention of the user may be prevented from being generated.

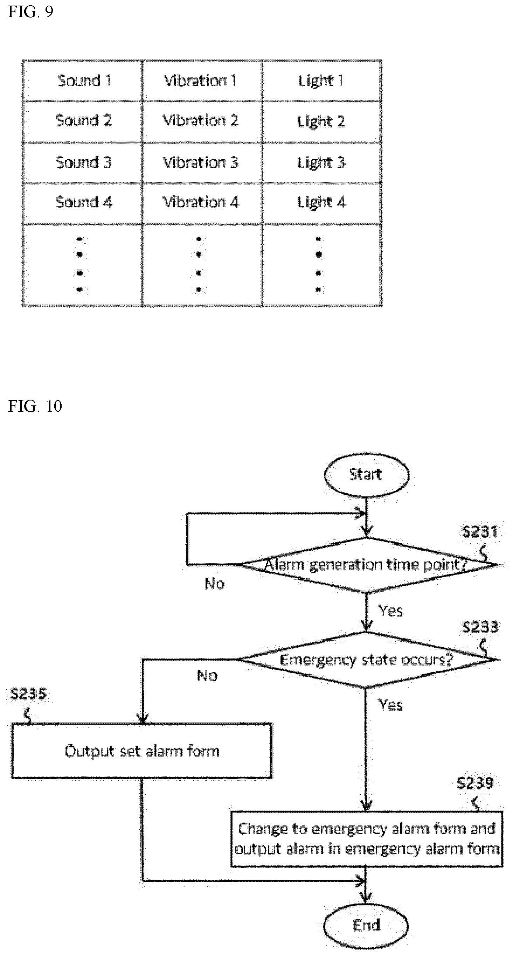

FIG. 9 illustrates an example of a mapping table at a time of changing an alarm form so that the alarm form conforms with an operation mode of a receiver.

As illustrated in FIG. 9, when the alarm form is changed to conform with the operation mode of the receiver, the alarm form is changed based on a mapping table.

For example, when an alarm form combination for a hypoglycemia alarm is formed of sound 4, sound 1, and sound 4, and when the operation mode of the receiver is set to be a vibration mode at a time of generating an alarm generation signal for sound 4, a first form alarm generation signal is generated with a change to vibration 4 mapped to sound 4 in the mapping table.

FIG. 10 is a flowchart for describing an example of providing an emergency alarm in a method of providing an alarm according to the present disclosure.

Here, the emergency alarm is an alarm for showing an emergency state such as a state in which a user is in acute hypoglycemia, a state in which a blood sugar change rate of the user is rapidly changed, or a state in which an estimated future blood sugar value is estimated to show acute hypoglycemia. The emergency alarm may be applied to various types of emergency states depending on a filed to which the present disclosure is applied, which is within the scope of the present disclosure.

In specific description with reference to FIG. 10, whether a current time is an alarm generation time point is determined in operation S231. Here, the alarm generation time point may be a time point of satisfying an alarm generation condition or a time point of sequentially generating an alarm generation signal for each alarm type according to an alarm form combination after the alarm generation condition is satisfied.

When the current time is the alarm generation time point, whether a state of the user is the emergency state is determined based on biometric data on the user in operation S233. Here, the state of the user may be determined as the emergency state when the biometric data which is determined at the alarm generation time point satisfies a first alarm generation condition, or when the biometric data which is determined at the alarm generation time point is a second alarm generation condition while a change rate of the biometric data exceeds a threshold change rate.

Here, the first alarm generation condition has a feature of being an alarm generation condition that the current biometric data shows acute hypoglycemia or is less than a predetermined value or that an estimated future blood sugar value shows acute hypoglycemia or a state of being less than the predetermined value.

As an example, the predetermined value has a feature of being adjustable by the user.

As another example, the predetermined value has a feature of being adjustable by a manufacturer. Here, the predetermined value may be predetermined by the manufacturer and may be allowed to be changed or not changed by the user.

Meanwhile, the second alarm generation condition is an alarm generation condition that the biometric data shows hypoglycemia or hyperglycemia, and the change rate of the biometric data has a feature of being a blood sugar change rate.

When the user is not in the emergency state at the alarm generation time point, the alarm generation signal is generated in an alarm form, which is set according to the alarm form combination for each set alarm type, and output in operation S235.

However, when the state of the user is the emergency state, in operation S239, an alarm form is changed to an emergency alarm form, and an emergency alarm generation signal is generated and output according to the changed emergency alarm form irrespective of an alarm form predetermined according to the alarm form combination for each alarm type or the operation mode of the receiver.

Here, the emergency alarm form may be an alarm generation signal providing an alarm with a strongest sound or an alarm generation signal for simultaneously providing different forms of alarms with the strongest sound.

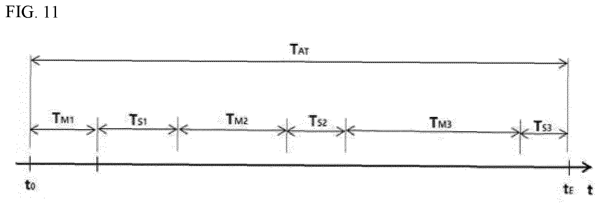

FIG. 11 is a diagram for describing an example of a generation condition, by alarm, for an emergency alarm pattern combination in the present disclosure.

As illustrated in FIG. 11, alarms in different forms are sequentially provided to a user in an alarm period TAT of the emergency alarm pattern combination. A first form alarm is generated to be a generation condition of a first activation time TM1 and a first stop time TS1, is output to the user for the first activation time TM1, and then stops being output for the first stop time TS1.

A second form alarm is generated to be a generation condition of a second activation time TM2 and a second stop time TS2, is output to the user for the second activation time TM2, and then stop being output for the second stop time TS2.

A third form alarm is generated to be a generation condition of a third activation time TM3 and a third stop time TS3, is output to the user for the third activation time TM3, and then stops being output for the third stop time TS3.

An activation time and a stop time of each alarm pattern forming the emergency alarm pattern combination may be equal to each other. However, desirably, depending on a generation order of alarm patterns, the activation time may be increased, and the stop time may be decreased. As such, by increasing the activation time and decreasing the stop time depending on the generation order of the alarm patterns, an alarm may be further accurately recognized as a time elapses for an alarm with a high degree of danger, or the user may be notified of urgency in a countermeasure with a lapse of a time.

Meanwhile, the above-described example embodiments may be prepared with a computer-executable program and implemented in a general-purpose digital computer for operating the program with a computer-readable recording medium.

The computer-readable recording medium includes a magnetic storage medium (e.g., a read-only memory, a floppy disk, a hard disk, or the like), an optical reading medium (e.g., a compact disc (CD)-ROM, a digital versatile disc (DVD), or the like), and a storage medium such as a carrier wave (e.g., transmission through the Internet).

## Claims

1. A method for providing an alarm for biometric data, the method comprising:
determining whether a set alarm generation condition is satisfied; and
based on that the alarm generation condition is satisfied, generating an alarm by modifying the alarm form during a set alarm period, and sequentially outputting the generated alarm to a user.

2. The method of claim 1, wherein the outputting of the alarm to the user comprises:
outputting a first form alarm by generating the first form alarm based on that the alarm generation condition is satisfied;
determining whether an alarm stop order is input or whether an alarm activation time for the first form alarm is ended;
stopping output of the first form alarm, which is currently output, based on that the alarm stop order is input or based on that the alarm activation time for the first form alarm is ended;
determining whether an alarm cancelation condition is satisfied after the output of the first form alarm is stopped; and
based on that the alarm generation condition is not satisfied, outputting, to the user, an alarm in a form by generating the alarm in the form is different from that of the first form alarm.

3. The method of claim 2, wherein the alarm in the changed form is generated to be sequentially output to the user before an alarm cancelation order is input in the alarm period.

4. The method of claim 2 or 3, wherein the alarm in the changed form is generated to be sequentially output to the user until the alarm cancelation condition is satisfied in the alarm period.

5. The method of any one of claims 2 to 4, wherein the alarm form which is sequentially changed in the alarm period is sequentially changed and generated depending on a predetermined alarm form.

6. The method of any one of claims 2 to 4, wherein the alarm form which is sequentially changed in the alarm period is randomly set and sequentially changed and generated.

7. The method of any one of claims 2 to 6, further comprising determining an operation mode of a receiver at a time point of generating the alarm,
wherein the alarm is generated and output in a form conforming with the operation mode of the receiver.

8. The method of any one of claims 2 to 6, wherein the method comprises determining, at a time point of generating the alarm, whether the alarm generation condition is a first alarm generation condition, and
based on that the alarm generation condition which is determined at the time point of generating the alarm is the first alarm generation condition, the generated alarm is output in a form of an emergency alarm disregarding the set alarm form or an operation mode of a receiver.

9. The method of claim 8, wherein the first alarm generation condition is an alarm generation condition that the biometric data shows acute hypoglycemia.

10. The method of claim 8 or 9, wherein the first alarm generation condition is that an estimated blood sugar value is in a state of being less than a predetermined value.

11. The method of claim 10, wherein the predetermined value is adjustable by the user.

12. The method of claim 10, wherein the predetermined value is not adjustable by the user.

13. The method of any one of claims 2 to 12, wherein whether the alarm generation condition is a second alarm generation condition and whether a change rate of the biometric data exceeds a threshold change rate are simultaneously determined at a time point of generating the alarm, and
based on that the change rate of the biometric data exceeds the threshold change rate while the alarm generation condition is the second alarm generation condition, the alarm is output in a form of an emergency alarm disregarding the set alarm form or an operation mode of a receiver.

14. The method of claim 13, wherein the second alarm generation condition is an alarm generation condition that the biometric data shows hypoglycemia or hyperglycemia.
